# EUROPEAN PATENT SPECIFICATION

(11) **EP 2 414 367 B1**
(45) Date of publication and mention of the grant of the patent: **13.08.2014**
(21) Application number: 10712850.6
(22) Date of filing: 01.04.2010
(51) Int. Cl.: C07D 493/08

(54) **PROSTAGLANDIN E RECEPTOR ANTAGONISTS**
ANTAGONISTEN DES PROSTAGLANDIN-E-REZEPTORS
ANTAGONISTES DU RÉCEPTEUR DE LA PROSTAGLANDINE E

(30) Priority: 02.04.2009 US 166107 P
(43) Date of publication of application: 08.02.2012
(73) Proprietor: ALLERGAN, INC., Irvine, CA 92612 (US)
(72) Inventor: WOODWARD, David, F., Lake Forest California 92630 (US); WANG, Jenny, W., Newport Coast California 92657 (US)
(74) Representative: HOFFMANN EITLE
(86) International application number: PCT/US2010/029626
(87) International publication number: WO 2010/114997

(56) References cited:
- US-A- 5 100 889
- US-A1- 2004 162 323
- US-A1- 2005 054 699
- NIRODI CHAITANYA S ET AL: "The glyceryl ester of prostaglandin E2 mobilizes calcium and activates signal transduction in RAW264.7 cells.", PROCEEDINGS OF THE NATIONAL ACADEMY OF SCIENCES OF THE UNITED STATES OF AMERICA, vol. 101, no. 7, 17 February 2004 (2004-02-17), pages 1840-1845, ISSN: 0027-8424

## Description

### RELATED APPLICATION

This application claims the benefit of U.S. Provisional Application Serial No. 61/166,107, filed April 2, 2009.

### BACKGROUND OF THE INVENTION

### FIELD OF THE INVENTION

The present invention provides PGE receptor antagonists, particularly, antagonists of the PGE₂-glyceryl ester receptor.

### DESCRIPTION OF RELATED ART

It would be desirable to have prostaglandin receptor antagonists to assist in pharmacologically defining prostaglandin receptors to aid in determining compounds which have activity at the individual prostaglandin receptors.

Prostaglandin F_{2α} antagonists are reported in U.S. Patent Nos. 4,632,928; 5,747,660; and 5,955,575. The PGF_{2α} antagonists of U.S. Patent No. 4,632,928 are pyrazole derivatives having an ergoline skeleton. The PGF_{2α} antagonist of U.S. Patent No. 5,747,660 is a prostaglandin F_{2α} receptor regulatory protein (FPRP) which is able to inhibit the binding of PGF_{2α} to its receptor.

Novel prostaglandin F2α antagonists are reported in U.S. Patent Nos. 6,369,089; 6,407,250; 6,509,364 and 6,511,999.

Prostaglandin receptor antagonists having EP4 and D2 activity are disclosed in published U.S. Patent Applications 20050065200 and 20040162323, respectively.

Interphenylene 7-Oxabicyclo [2.2.1] heptane oxazoles, useful as Thromboxane A₂ receptor antagonists are reported in U.S. Patents 5,100,889 and 5,153,327, European Patent Application 0 391 652 and J. Med. Chem. 1993, 36, 1401-1417.

Thromboxane A₂ receptor antagonists, e.g. 7-oxabicycloheptyl substituted heterocyclic amide prostaglandin analogs, alone, or in combination with anti-inflammatory agents are useful in treating ulcerative gastrointestinal conditions and dysmenorrhea as disclosed in European Patent Application 0 448 274 and U.S. Patent 5,605,917.

U.S. Patent Application 2005 0054 699 discloses prostamide F_{2α} receptor antagonists.

### BRIEF SUMMARY OF THE INVENTION

The invention relates to prostaglandin receptor antagonists, e.g. prostaglandin E receptor antagonists and their use in determining compounds having agonist activity at the prostaglandin E receptor including subtypes thereof, i.e. prostaglandin E4 receptor antagonists and said compounds for use in the treatment of various diseases and conditions in humans, e. g. bone-related diseases.

The compounds useful as prostaglandin E receptor antagonists of the present invention may be represented by the general formula I. 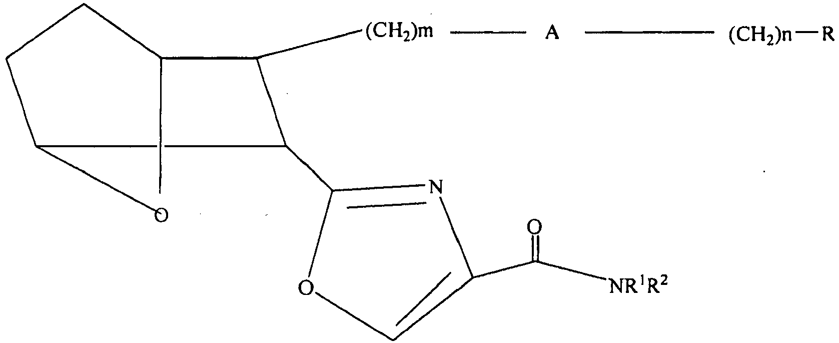 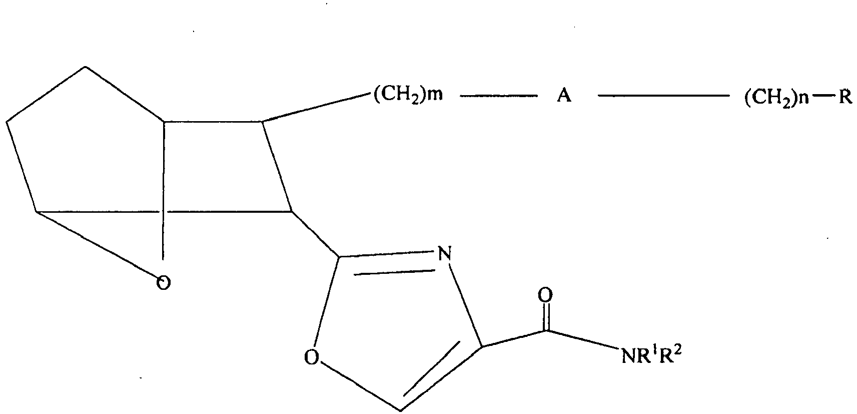
wherein R is C(O)R³-CH₂-CH(OH)(CH₂OH)- or C(O)R³-CH(CH₂OH)₂ and R³ is selected from the group consisting of O, NR⁴, S or C(R⁵)₂
wherein R⁴ represents a radical selected from the group consisting of H, hydrocarbyl and heteroatom-substituted hydrocarbyl radicals, wherein said hetero atom is selected from the group consisting of halogen, O, S and N, i.e. O, S and/or N may be included as a O, S, or N-containing radical, e.g. R⁴ is H, alkyl, alkenyl, alkynyl or aryl, or heteroatom-substituted alkyl, alkenyl, alkynyl or aryl (heteroaryl) radical and R⁵ represents a radical selected from the group consisting of H, hydrocarbyl and heteroatom-substituted hydrocarbyl radicals, wherein said hetero atom is selected from the group consisting of halogen, O, S and N, i.e. O, S and/or N may be included as a O, S, or N-containing radical, e.g. H, alkyl, alkenyl, alkynyl and aryl, or heteroatom-substituted alkyl, alkenyl, alkynyl and aryl (heteroaryl) radicals;
m is an integer of from 1 to 3, preferably 1 or 2;
n is 0 or an integer of from 1 to 4, preferably from 2 to 4;
A is an aryl or heteroaryl radical having from 6 to 14 carbon atoms, wherein said heteroaryl may be substituted with one or more oxygen, sulfur or nitrogen in the heteroaryl ring and heteroatom substituted derivatives thereof;
R¹ and R² are independently selected from the group consisting of H, C₁-C₆ alkyl, C₂-C₆ alkenyl, C₂-C₆ alkynyl, C₃-C₇ cycloalkyl, C₄-C₁₂ alkylcycloalkyl, C₆-C₁₀ aryl, C₇-C₁₂ alkylaryl radicals and heteroatom-substituted derivatives thereof, wherein one or more of the hydrogen or carbon atoms in said radicals may be replaced with a halogen, oxygen, nitrogen or sulfur-containing radical; and pharmaceutically acceptable salts thereof.

The preferred substituents for R¹ and R² are selected from the group consisting of hydroxyl, halogen, e.g. fluoro or chloro, COOR⁶, NO₂, N(R⁶)₂, SR⁶, sulfoxy, sulfone, CN and OR⁶, wherein R⁶ is H or C₁-C₆ alkyl.

These compounds are especially useful for determining compounds having prostaglandin E agonist activity, as well as for treating a number of diseases. PGE2 is known as one of the metabolites in an arachidonate cascade. And it is also known that it has various activities such as pain inducing activity, inflammatory activity, uterine contractile activity, a promoting effect on digestive peristalsis, an awaking activity, a suppressive effect on gastric acid secretion, hypotensive activity, blood platelet inhibition activity, bone-resorbing activity, angiogenic activity, or the like.

Prostaglandin E-sensitive or PGE2 -sensitive receptors have been sub-divided into four subtypes, EP1, EP2, EP3 and EP4, and these receptors have a wide distribution in various tissues. The effects associated with EP1 and EP3 receptors may be considered as excitatory, and are believed to be mediated by stimulation of phosphatidylinositol turnover or inhibition of adenyl cyclase activity, with resulting decrease in intracellular levels of cyclic AMP. In contrast, the effects associated with EP2 and EP4 receptors may be considered as inhibitory, and are believed to be associated with a stimulation of adenyl cyclase and an increase in levels of intracellular cyclic AMP. Especially, EP4 receptor may be considered to be associated with smooth muscle relaxation, anti-inflammatory or pro-inflammatory activities, lymphocyte differentiation, antiallergic activities, mesangial cell relaxation or proliferation, gastric or enteric mucus secretion, or the like.

The compounds represented by the formula (I) or its salts possess binding activities to PGE2 -sensitive receptors, therefore they possess a PGE2 -antagonizing or PGE2 - inhibiting activity.

Therefore, the compounds represented by the formula (I) or its salts are useful for preventing or treating a PGE2 mediated diseases, such as blocking PGE2-glyceryl ester effects, such as inflammatory conditions, various pains, or the like in human beings or animals.

More particularly, the compounds represented by formula (I) and its salt are useful for treating or preventing inflammation and pain in joint and muscle (e.g., rheumatoid arthritis, rheumatoid spondylitis, osteoarthritis, gouty arthritis, juvenile arthritis, etc.), inflammatory skin condition (e.g., sunburn, burns, eczema, dermatitis, etc.), inflammatory eye condition (e.g., conjunctivitis, etc.), lung disorder in which inflammation is involved (e.g., asthma, bronchitis, pigeon fancier's disease, farmer's lung, etc.), condition of the gastrointestinal tract associated with inflammation (e.g., aphthous ulcer, Chrohn's disease, atopic gastritis, gastritis varialoforme, ulcerative colitis, coeliac disease, regional ileitis, irritable bowel syndrome, etc:), gingivitis, inflammation, pain and tumescence after operation or injury, pyrexia, pain and other conditions associated with inflammation, allergic disease, systemic lupus erythematosus, scleroderma, polymyositis, tendinitis, bursitis, periarteritis nodose, rheumatic fever, Sjogren's syndrome, Behcet disease, thyroiditis, type I diabetes, diabetic complication (diabetic microangiopathy, diabetic retinopathy, diabetic neohropathy, etc.), nephrotic syndrome, aplastic anemia, myasthenia gravis, uveitis, contact dermatitis, psoriasis, Kawasaki disease, sarcoidosis, Hodgkin's disease, Alzheimers disease, kidney dysfunction (nephritis, nephritic syndrome, etc), liver dysfunction (hepatitis, cirrhosis, etc.), gastrointestinal dysfunction (diarrhea, inflammatory bowel diseases, etc.) shock, bone disease characterized by abnormal bone metabolism such as osteoporosis (especially, postmenopausal osteoporosis), hyper-calcemia, hyperparathyroidism, Paget's bone diseases, osteolysis, hypercalcemia of malignancy with or without bone metastases, rheumatoid arthritis, periodontitis, osteoarthritis, ostealgia, osteopenia, cancer cachexia, calculosis, lithiasis (especially, urolithiasis), solid caricinoma, or the like in human being or animal. For instance, PGE₂ antagonists may be useful in treating hyperpigmentary disorders of the skin, hair, internal organs or other pigmented cells. Additionally, prostaglandin antagonists may be useful in reducing hair growth, e.g. in case of hirsutism or in instances where a reduction or prevention of hair growth may be desirable. Also, prostaglandin antagonists may be useful in treating ocular hypotony associated with disease or surgery. Finally, prostaglandin antagonists may be useful in treating inflammatory and auto-immune diseases such as, but not limited to, reheumatoid arthritis, uveitis, and conjunctivitis.

### BRIEF DESCRIPTION OF THE DRAWING FIGURES

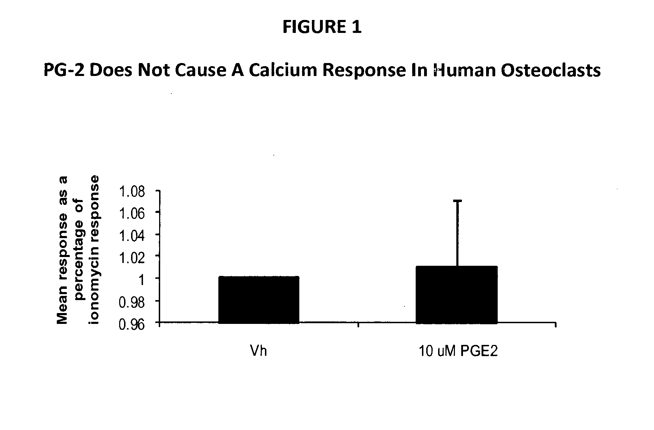

Figure 1 shows that PG-2 does not cause a Calcium Response in human osteoclasts;

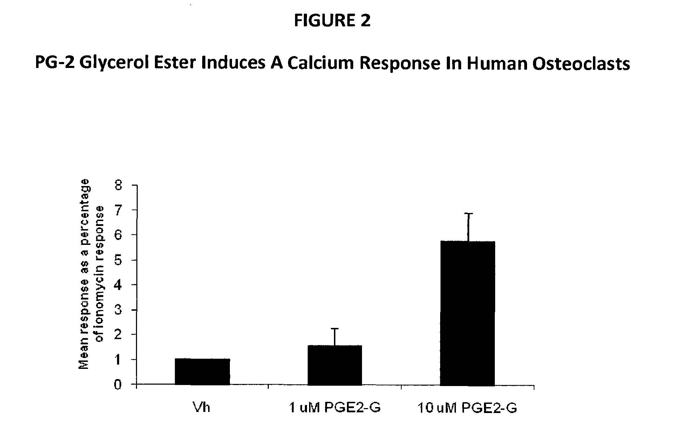

Figure 2 shows that PG-2 Glycerol Ester induces a Calcium Response in human osteoclasts;

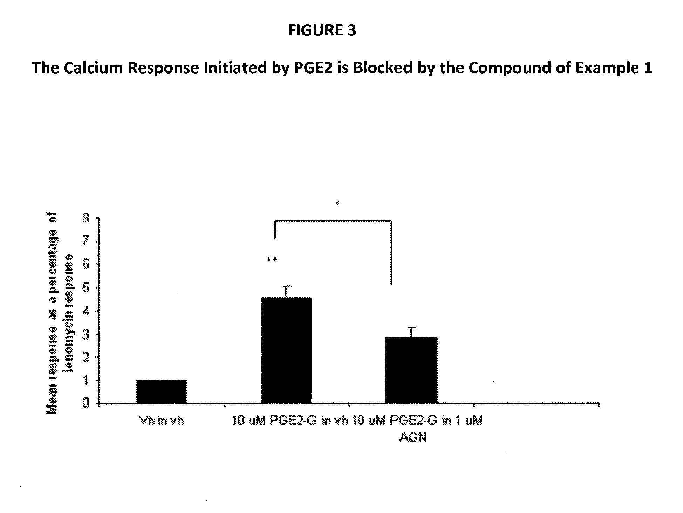

Figure 3 shows that the Calcium Response initiated by PGE2 is blocked by the Compound of Example 1;

### DETAILED DESCRIPTION OF THE INVENTION

In the PGE₂ receptor antagonists, e.g. antagonists of EP4 or the PGE₂-glyceryl ester-specific receptor described below, of the present invention, A may be represented by the general formula 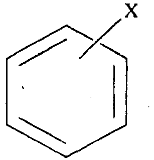 or 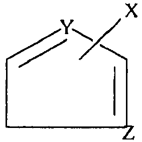
wherein X is selected from the group consisting of H, R⁶, hydroxy, halogen, e.g. fluoro or chloro, COOR⁶, NO₂, CF₃,
N(R⁶)₂, CON(R⁶)₂, SR⁶, sulfoxy, sulfone, CN and OR⁶ wherein R⁶ is H or C₁-C₆ alkyl; Y is O or S; Z is N or CH

Preferably, the prostaglandin antagonist compounds are represented by the general formula II. 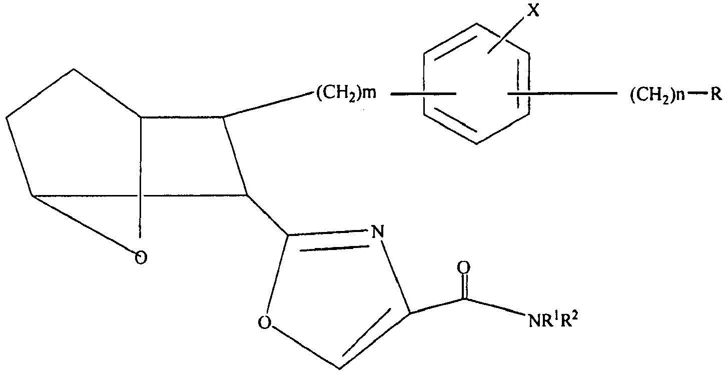 or general formula III 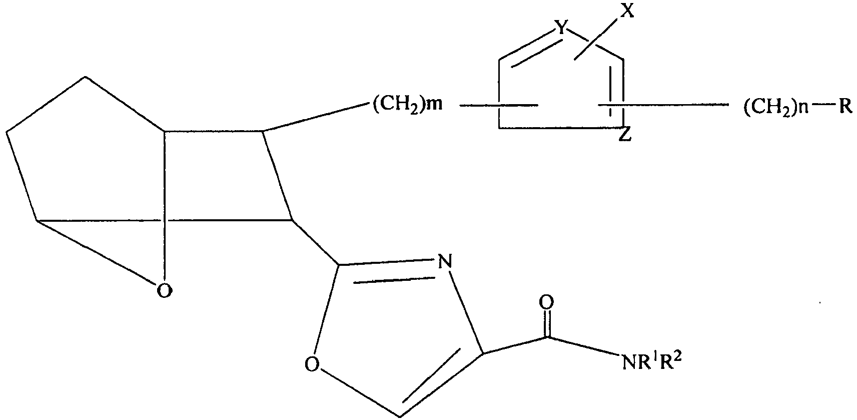 wherein R is defined above.

Preferably, R¹ and R² are selected from the group consisting of H, C₁-C₆alkyl, C₃-C₇ cycloalkyl and C₄-C₁₂ alkylcycloalkyl.

Preferably, R⁴ and R⁵ are selected from the group consisting of H and C₁-C₆ alkyl. Most preferably R⁴ and R⁵ are H.

Preferably, X is selected from the group consisting of hydrogen or halogen, e.g. fluoro.

Preferably, R is C(O)NH-CH₂-CH(OH)(CH₂OH) or C(O)NH-CH(CH₂OH)₂

The most preferred compounds may be described as the serinolamide or dihydroxypropylamide derivative of 3-(2-{(1R,2R,3S,4R)-3-[4-(4-Cyclohexyl-butylcarbamoyl)-oxazol-2-yl]-7-oxa-bicyclo[2.2.1]hept-2-ylmethyl}-4-fluoro-phenyl)-propionic acid , i.e. wherein the 1-OH is replaced with the serinolamide or dihydroxypropylamide, respectively.

The following definitions may be used throughout this specification.

"Pharmaceutically acceptable salt" refers to those salts which retain the biological effectiveness and properties of the free bases and which are obtained by reaction with inorganic acids such as hydrochloric acid, hydrobromic acid, sulfuric acid, nitric acid, phosphoric acid, methanesulfonic acid, ethanesulfonic acid, p-toluenesulfonic acid, salicylic acid and the like. Pharmaceutically acceptable salt" may also refer to those salts which retain the biological effectiveness and properties of the free acid and which are obtained by reaction with inorganic bases such as sodium hydroxide, potassium hydroxide or calcium hydroxide and the like or organic bases such as lysine, arginine, ethanolamine and the like.

"Alkyl" refers to a straight-chain, or branched saturated aliphatic hydrocarbon. Preferably, the alkyl group has 1 to 12 carbons. More preferably, it is a lower alkyl of from 1 to 6 carbons, most preferably 1 to 4 carbons. Typical alkyl groups include methyl, ethyl, propyl, isopropyl, butyl, isobutyl, tertiary butyl, pentyl, hexyl and the like.

"Alkenyl" refers to a straight-chain, branched or cyclic unsaturated hydrocarbon group containing at least one carbon-carbon double bond. Preferably, the alkenyl group has 1 to 12 carbons. More preferably it is a lower alkenyl of from 1 to 7 carbons, most preferably 1 to 4 carbons.

"Alkynyl" refers to a straight-chain, branched or cyclic unsaturated hydrocarbon containing at least one carbon-carbon triple bond. Preferably, the alkynyl group has 1 to 12 carbons. More preferably it is a lower alkynyl of from 1 to 7 carbons, most preferably 1 to 4 carbons.

"Alkoxy" refers to an "O-alkyl" group.

"Aryl" refers to an aromatic group which has at least one ring having a conjugated pi electron system and includes carbocyclic aryl, heterocyclic aryl and biaryl groups.

"Alkaryl" refers to an alkyl that is covalently joined to an aryl group. Preferably, the alkyl is a lower alkyl.

"Carbocyclic aryl" refers to an aryl group wherein the ring atoms are carbon.

"Heterocyclic aryl or heteroaryl" refers to an aryl group having from 1 to 3 heteroatoms as ring atoms, the remainder of the ring atoms being carbon.

"Heteroatoms" include oxygen, sulfur, and nitrogen. Thus, heterocyclic aryl groups include furanyl, thienyl, pyridyl, pyrrolyl, N-lower alkyl pyrrolo, pyrimidyl, pyrazinyl, imidazolyl and the like.

"Hydrocarbyl" refers to a hydrocarbon radical having only carbon and hydrogen atoms. Preferably, the hydrocarbyl radical has from 1 to 20 carbon atoms, more preferably from 1 to 12 carbon atoms and most preferably from 1 to 6 carbon atoms.

"Heteroatom substituted hydrocarbyl" refers to a hydrocarbyl radical wherein one or more, but not all, of the hydrogen and/or the carbon atoms are replaced by a halogen, nitrogen, oxygen, or a sulfur atom or a radical including a halogen, nitrogen, oxygen, or sulfur atom, e.g. fluoro, chloro, cyano, nitro, hydroxyl, phosphate, thiol, etc.

"Amide" refers to -C(O)-NH-R', wherein R' is alkyl, aryl, alkylaryl or hydrogen.

"Amine" refers to a -N(R")R"' group, wherein R" and R"' are independently selected from the group consisting of alkyl, aryl, and alkylaryl.

THF refers to tetrahydrofuran.

DCM refers to dichloromethane

DIBAL-H refers to diisobutylaluminumhydride

DMAP refers to 4-dimethylaminopyridine

The following Examples describe a method of synthesizing the prostaglandin antagonist compounds of the invention wherein the numbering of the Examples corresponds to the numbering of the various intermediates and final compounds shown in the reaction scheme described in US Patent Number 7,217,725.

### Example 1

SYNTHESIS OF *N*-(1,3-Dihydroxyprop-2-yl)-3-(2-{(1R,2R,3S,4R)-3-[4-(4-cyclohexyl-butylcarbamoyl)-oxazol-2-yl]-7-oxa-bicyclo[2.2.1]hept-2-ylmethyl}-4-fluoro-phenyl)-propionamide.

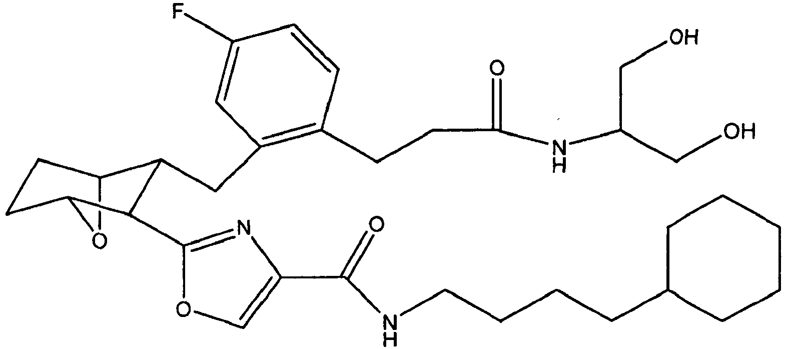

### SYNTHESIS OF LACTOL INTERMEDIATE 10

### (1) Chiral monomenthol ester

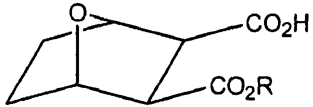

CO₂R = *L*-Menthol ester

To a stirred solution of *L*-menthol (202g: 1.3moles) in anhydrous THF (1 L) at 0°C was added *n*-butyllithium (2.5M in hexanes; 510m; 1.28moles) keeping the temperature below 10°C. The reaction was now cooled to -78°C whereupon a solution of meso-7-oxabicyclo[2.2.1]heptane-2,3-dicarboxylic anhydride (177g; 1.05moles) in anhydrous THF (1.2L) was added over *circa* 20 minutes keeping the temperature below -50°C. After addition, the reaction was left at -64°C for a further hour then quenched with dilute hydrochloric acid (2M; 800ml) over 5 minutes, causing the temperature to rise to *circa -* 25°C. Brine (400ml) was added and the layers separated. The organic layer was washed with brine (400ml). The combined aqueous layers were re-extracted with DCM (1L). The combined organic layers were dried over sodium sulfate and evaporated *in vacuo.* The residue was dissolved in a mixture of DCM (700ml) and ethyl acetate (2800ml) upon boiling. The solution was cooled slowly to room temperature, left at 4°C for 21 hours, filtered, washed with ethyl acetate (2x500ml) and dried *in vacuo* to yield the monomenthol ester as the pure desired diastereomer (chiral purity confirmed by NMR analysis; 111.5g; 66%).

### (2) Chiral Lactone

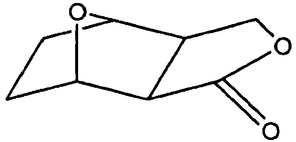

To a 0°C solution of the monomenthol ester (65g; 0.20moles) and triethylamine (freshly distilled ex CaH₂; 55ml) in anhydrous THF (650ml) was added ethyl chloroformate (37ml; 0.39moles), keeping the temperature below 10°C. After addition the reaction mixture was left at 0°C for an hour then ether (700ml; previously dried over molecular sieves) and petroleum ether (700ml) were added. The mixture was filtered through magnesium sulfate (200g) and washed through with ether (2x400ml). The filtrate was *evaporated in vacuo.* The white solid residue was dissolved in THF (550ml), cooled to 5°C, absolute alcohol (800ml) added, then sodium borohydride (15g; 0.39moles) was added portionwise over 2 minutes. After 15 minutes dilute hydrochloric acid (2M; 600ml) and ice were added and the mixture extracted with DCM (3x750ml). The combined organic layers were dried over sodium sulfate and evaporated *in vacuo.* The oily residue was re-dissolved in DCM (600m1), *para*-toluenesulfonic acid monohydrate (3.75g; 0.02moles) added and the mixture was stirred for 25 minutes. After washing with sodium bicarbonate solution (200ml), the organic layer was dried over sodium sulfate and evaporated *in vacuo*. The residue was boiled in petroleum ether (40-60°C; 300ml), cooled to -20°C, filtered and washed with petroleum ether (3x50ml) to afford the chiral lactone (24.5g; 79%).

### (3) Chiral Lactol Intermediate 10

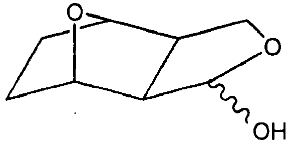

DIBAL-H (25% in toluene; 170ml) was added over 40 minutes to a solution of the lactone (25g; moles) in anhydrous toluene (400ml) at -78°C. The temperature was kept below -60°C during addition. After a further 30 minutes acetic acid (50ml) was added cautiously dropwise. The reaction mixture was warmed to room temperature then added to dilute hydrochloric acid (2M; 250ml). The toluene layer was separated and the aqueous layer saturated with sodium chloride and extracted with DCM (4x600ml). The combined organic layers were dried over sodium sulfate, evaporated *in vacuo* and azeotroped with toluene (2x300ml) to afford **Intermediate 10** (25g; 99%).

### SYNTHESIS OF ARYLBROMIDE INTERMEDIATE 8

(4) To a suspension of methyl (triphenylphosphoranylidene)acetate (233.3g; 0.70moles) in anhydrous THF (1.2L) was added a solution of 2-bromo-4-fluorobenzaldehyde (**Intermediate 4;** 141.6g; 0.70 moles) in anhydrous THF (150my) dropwise over 30 minutes. The mixture was stirred overnight and the solvent removed *in vacuo.* The residue was triturated in petroleum ether (b.p. 40-60°C; 1 L) and passed through silica (1Kg), eluting with 10% ethyl acetate in petroleum ether to afford **Intermediate 5** (176g; 97%) as a mixture of *cis* and *trans* isomers.

(5) A mixture of **Intermediate 5** (60g; 0.23moles) and 5% rhodium on carbon (10g) in THF (600ml) was stirred under hydrogen for 2 days at room temperature. The mixture was filtered through celite and the filter pad washed with THF (3x200ml). The filtrate was *evaporated in vacuo* to yield **Intermediate 6** (60g; 99%).

(6) To a -70°C solution of **Intermediate 6** (26.1g; 0.10mol in anhydrous toluene (150ml) was added DIBAL-H (25% in toluene; 140ml) over 20 minutes, keeping the temperature below -50°C. The mixture was stirred at -60°C for a further 30 minutes then at 0°C for a further hour. Hydrochloric acid (6M; 150ml) was added cautiously keeping the temperature below 40°C. The organic layer was separated and the aqueous layer extracted with toluene (2x50ml). The combined organic extracts were dried over sodium sulfate and *evaporated in vacuo* to afford **Intermediate 7** (23.3g; 100%).

(7) To a solution of **Intermediate 7** (81.5g; 0.35moles), triethylamine (56ml) and DMAP (1.75g; 0.014moles) in anhydrous DCM (700ml) was added dimethylthexylsilyl chloride (70ml; 0.355moles) dropwise over 20 minutes. The mixture was left overnight at room temperature. Water (200ml) was added and the layers separated. The aqueous layer was extracted with dichloromethane (2x100ml). The combined organic layers were dried over sodium sulfate and passed through silica (500g), eluting with DCM to yield **Intermediate 8** (125g; 95%).

### GRIGNARD COUPLING OF INTERMEDIATES 8 AND 10 AND ELABORATION TO N-(1,3-Dihydroxyprop-2-yl)-3-(2-{(1R,2R,3S,4R)-3-[4-(4-cyclohexyl-butylcarbamoyl)-oxazol-2-yl]-7-oxa-bicyclo[2.2.1]hept-2-ylmethyl}-4-fluoro-phenyl)-propionamide

(8) A catalytic quantity of iodine was added to a mixture of magnesium turnings (15g; 0.62moles) and aryl bromide **Intermediate 8** (140g; 0.37moles) in anhydrous THF (500ml). After the initial reaction had subsided the mixture was heated to 60°C for 2 hours to complete formation of the Grignard compound **Intermediate 9** then cooled to room temperature.

Ethyl magnesium bromide (1M in THF; 280ml; 0.28moles) was added dropwise over 15 minutes at 0-5°C to a solution of **Intermediate 10** (45g; 0.29moles) in anhydrous THF (260ml). The solution was left at 0-5°C for a further 25 minutes then the prepared Grignard solution **Intermediate 9** (described above) was added *via* cannula over 10 minutes. The reaction mixture was warmed to room temperature and stirred for 24 hours, concentrated *in vacuo* and partitioned between DCM (2L) and ammonium chloride solution (1L). Dilute hydrochloric acid (2M) was added to the stirred mixture to reduce the pH to 8-9. The organic layer was separated, dried over sodium sulfate and evaporated *in vacuo*. Chromatography of the residue in 5-40% ethyl acetate in hexanes afforded the product **Intermediate 11** (111 g; 88%).

(9) A solution of Grignard product **Intermediate 11** (111g; 0.25moles) and acetic anhydride (28ml; 0.30moles) in anhydrous pyridine (200ml) was stirred overnight at ambient temperature then evaporated *in vacuo*. The residue was dissolved in hexanes (1.2L), washed with water (500ml), dilute hydrochloric acid (2x500ml), water (500ml), brine (500ml), dried over sodium sulfate and *evaporated in vacuo* to give the product **Intermediate 12** (130g; minor amounts of diacetate/solvent present) which was used in the next step without further purification.

(10) A mixture of crude **Intermediate 12** (38g) and 10% palladium on carbon (10g) in acetic acid (200ml) was stirred under hydrogen for 40 hours at 55°C. After cooling, ethyl acetate (200ml) was added, the mixture was filtered through celite and the filter pad washed through with ethyl acetate (3x200ml). The filtrate was evaporated *in vacuo* and the residue chromatographed in 10% ethyl acetate in hexanes to elute firstly the de-hydroxylated material **Intermediate 12a** (14.5g; 0.030moles) then eluting with ethyl acetate to afford the de-silylated de-hydroxylated material **Intermediate 12b** (6.5g; 0.019moles). Yield over two steps from **Intermediate 11:** 68%. These two products (illustrated below) were combined for the next reaction.

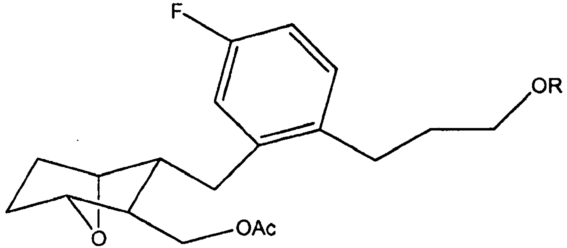

### Intermediate 12a: R = dimethylthexylsilyl

### Intermediate 12b: R = H

(11) Jones' reagent (35ml) was added to a water-cooled solution of the above mixture of **Intermediate 12a** and **Intermediate 12b** (21 g; 0.049moles combined) in acetone (265ml). After 30 minutes 2-propanol (25ml) was added and the resultant mixture stirred for 15 minutes, filtered through celite and washed through with acetone. The filtrate was evaporated *in vacuo,* dissolved in DCM (500ml) and washed with water (200ml). The organic layer was dried over sodium sulfate and evaporated *in vacuo*. The residue was dissolved in anhydrous methanol (100ml) to which was added acetyl chloride (2ml) and stirred overnight. After evaporation, the residue was chromatographed in 60% ethyl acetate in hexanes to yield the product **Intermediate 15** (9.3g; 59%).

(12) Jones' reagent (24ml) was added to a water-cooled solution of **Intermediate 15** (10.2g; 0.032moles) in acetone (260ml). After 35 minutes 2-propanol (16ml) was added and the resultant mixture stirred fir 15 minutes, filtered through celite and washed through with acetone. The filtrate was evaporated *in vacuo*, dissolved in DCM (500ml) and washed with water (200ml). The organic layer was dried over sodium sulfate and evaporated *in vacuo* to yield the acid-ester product **Intermediate 16** (10.2g; 95%) which was used in the next step without further purification.

(13) To a 0°C mixture of **Intermediate 16** (10.7g; 0.032moles), L-serine benzyl ester hydrochloride (8.8g; 0.038moles) and 1-hydroxybenzotriazole (6.3g; 0.046moles) in anhydrous THF (150m) was added triethylamine (11.5ml). After 5 minutes dicyclohexylcarbodiimide (1M in DCM; 45ml; 0.045 moles) was added, and the reaction mixture left at room temperature overnight. After concentrating *in vacuo*, the residue was slurried in ethyl acetate (200ml) and filtered. The filter pad was washed through with ethyl acetate (3x50ml) and the filtrate was evaporated *in vacuo*. The residue was chromatographed in 60-100% ethyl acetate in hexanes to yield **Intermediate 17** as a white solid (13g; 79%).

(14) To a 0°C solution of **Intermediate 17** (13g; 0.025moles), triphenylphosphine (19.5g; 0.074moles) and diisopropylethylamine (12.9ml; freshly distilled ex CaH₂; 0.074moles) in anhydrous DCM (24ml) and anhydrous acetonitrile (120ml) was added carbon tetrachloride (7.22ml; 0.074moles). The mixture was left at room temperature for 4 hours, cooled to 0°C and ethyl acetate (300ml) and sodium bicarbonate solution (300ml) were added. After stirring vigorously for 5 minutes, the mixture was added to ethyl acetate (500ml) and brine (400ml). The organic layer was separated, dried over sodium sulfate and evaporated *in vacuo*. The residue was chromatographed in 60% ethyl acetate in hexanes to yield **Intermediate 18** (6.1 g; 49%) as a pale cream solid.

(15) To a -10°C solution of **Intermediate 18** (6.1g; 0.012moles) in anhydrous DCM (60ml) was added DBU (2ml) followed by bromotrichloromethane (1.5ml). The reaction mixture was stoppered, left in the fridge overnight then washed with ammonium chloride solution (150ml). The aqueous layer was separated and back-extracted with DCM (100ml). The combined organic layers were dried over sodium sulfate and evaporated *in vacuo*. The residue was chromatographed in 40% ethyl acetate in hexanes to afford the oxazole **Intermediate 19** (4.7g; 78%).

(16) A mixture of **Intermediate 19** (1.8g; 3.65mmoles) and palladium hydroxide (20%; 0.5g) in ethyl acetate (35ml) was stirred under hydrogen for 2 hours. After cooling, the mixture was filtered through celite and the filter pad washed through with ethyl acetate (3x25ml). The filtrate was evaporated *in vacuo* to yield the product **Intermediate 20** (1.45g; 99%) as a white solid.

(17) Oxalyl chloride (0.91g; 0.61ml; 7.15mmoles) was added to a solution of **Intermediate 20** (1.45g; 3.60mmoles) in anhydrous DCM (15ml). One drop of *N,N-*dimethylformamide was added to catalyse the reaction. After 20 minutes, the mixture was *evaporated in vacuo,* azeotroped with anhydrous toluene (50ml) then dissolved in anhydrous dichloromethane (18ml) and cooled to 0°C. Triethylamine (freshly distilled ex CaH₂; 1.15ml) and 4-cyclohexylbutylammonium chloride (1g; 5.22mmoles) were added then the mixture was warmed to room temperature for 1.5 hours. The mixture was added to dilute hydrochloric acid (1M; 50ml) and extracted with DCM (3x50ml). The combined organic layers were dried over sodium sulfate and evaporated *in vacuo*. The residue was chromatographed in 50% ethyl acetate in hexanes to afford **Intermediate 21** (1.45g; 75%).

(18) To a solution of **Intermediate 21** (8.8g; 16.30mmoles) in THF (50ml) and methanol (230ml) was added sodium hydroxide solution (1M; 90ml). After 3 hours, acetic acid (6.5ml) was added and the solution concentrated to *circa* 100m *in vacuo*, dissolved in ethyl acetate (1L), washed with dilute hydrochloric acid (2M; 600ml), brine (300ml), dried over sodium sulfate and evaporated *in vacuo.* The residue was dissolved in DCM (70ml) and petroleum ether (40-60°C; 350ml) was added slowly to the warmed and stirred solution. The crystallizing mixture was cooled to room temperature over 20 minutes, filtered, washed with pentane (2x50ml) and dried *in vacuo* to afford *N*-(1,3-Dihydroxyprop-2-yl)-3-(2-{(1R,2R,3S,4R)-3-[4-(4-cyclohexyl-butylcarbamoyl)-oxazol-2-yl]-7-oxa-bicyclo[2.2.1]hept-2-ylmethyl}-4-fluoro-phenyl)-propionamide as a white solid (7.60g; m.p.165-166°C; 87%). The mother liquors were evaporated *in vacuo*, chromatographed in 5% methanol in dichloromethane and crystallized as above to give a further crop of *N*-(1,3-Dihydroxyprop-2-yl)-3-(2-{(1R,2R,3S,4R)-3-.[4-(4-cyclohexyl-butylcarbamoyl)-oxazol-2-yl]-7-oxa-bicyclo[2.2.1]hept-2-ylmethyl}-4-fluoro-phenyl)-propionamide (0.49g; m.p.165-166°C; 5.7%).

### Example 2

By substituting the appropriate reagent or intermediate the corresponding propionic acid or oxa analogue is prepared.

### Example 3

By substituting the appropriate reagent or intermediate the corresponding thio acid or sulfide analogue is prepared.

### Example 4

By substituting the appropriate reagent or intermediate the corresponding methylene analogue is prepared.

### Examples 5 through 8

By substituting the appropriate reagent or intermediate in the methods of synthesis of Examples 1 through 4 the corresponding dihydroxypropyl derivatives are prepared.

The compounds of the present invention are especially useful in treating diseases and conditions of bone in mammals, e.g. humans.

An intricate balance between the activities of two major cell types referred to as osteoblasts and osteoclasts determine a human's total bone mass.

Bone remodeling starts with resorption, which the osteoclasts orchestrate. Osteoclasts break down bone by dissolving mineral and resorbing the matrix that osteoblasts have formed.

Osteoblasts make collagen and hydroxyapatite. Some of the osteoblasts become buried in their matrix and then they are referred to as *osteocytes.* The rest of the osteoblasts cover the new bone's surface. Waves of osteoblasts that move into the area form new layers of bone.

Osteoclasts are larger cells whose function is to dissolve bone by acting on the mineral matrix. They make enzymes such as *collagenase,* which breaks down collagen. Osteoclasts also secrete various acids that can dissolve the hydroxyapatite structure.

There are a variety of signals that control the function of osteoblasts and osteoclasts. Osteoblasts make small proteins, one of which is OPG (osteoprotegrin). OPG can prevent osteoclasts from being activated.

Osteoblasts change their shape and become buried in their matrix, connected to each other only by thin processes called *canaliculi*. After the osteoblasts are buried in bone, they're referred to as osteocytes. Osteocytes account for 90 percent of all cells in the skeleton.

It is understood that the process of building up bone and resorption of bone is critical because abnormalities in these processes lead to bone diseases.

In particular, Accelerated osteoclastic bone resorption has a central role in the pathogenesis of osteoporosis and other bone diseases. Identifying the molecular pathways that regulate osteoclast activity provides a key to understanding the causes of these diseases and to the development of new treatments. It has been shown that mice with inactivation of cannabinoid type 1 (CB1) receptors have increased bone mass and are protected from ovariectomy-induced bone loss. Pharmacological antagonists of CB1 and CB2 receptors prevent ovariectomy-induced bone loss in vivo and cause osteoclast inhibition in vitro by promoting osteoclast apoptosis and inhibiting production of several osteoclast survival factors. Thus, the CB1 receptor has a role in the regulation of bone mass and ovariectomy-induced bone loss and CB1- and CB2-selective cannabinoid receptor antagonists are osteoclast inhibitors that are useful in the treatment of osteoporosis and other bone diseases.

The prostaglandin E₂ antagonists of the present invention may be used to test for compounds having prostaglandin E₂ glyceryl receptor agonist activity and not activity at the corresponding prostaglandin E₂ receptor as follows:

A tissue or cell responsive to a prostaglandin E₂-glyceryl ester and prostaglandin E₂, e.g. cat iris sphincter tissueXX, is contacted with various concentrations of said prostaglandin E-glyceryl ester and a first response is measured in a concentration dependent manner. (Preferably, the cat iris sphincter tissue may be dissected into four paired preparations for the purpose of the following test.) Said tissue or cell is contacted with said various concentrations of said prostaglandin E₂-glyceryl ester in the presence of a prostaglandin antagonist and a second response is measured in a concentration dependent manner.

Said tissue or cell is contacted with various concentrations of a compound which is to be evaluated for prostaglandin E₂-glyceryl ester agonist activity and a third response is measured in a concentration dependent manner. Said tissue or cell is contacted with said various concentrations of said compound which is to be evaluated for prostaglandin E₂-glyceryl ester agonist activity in the presence of said prostaglandin E₂-glyceryl ester antagonist and a fourth response is measured in a concentration dependent manner.

Compounds having prostaglandin E₂-glyceryl ester agonist activity are determined as compounds wherein the difference between said third and fourth response is greater than the difference between said first and second response.

Preferably, the difference between said first and second response is substantially negligible, i.e. the prostaglandin E₂-glyceryl ester has substantially no prostaglandin agonist activity, therefore the presence of the prostaglandin E₂-glyceryl ester antagonist does not affect the tissue response. Thus, prostaglandin E₂-glyceryl ester agonists are compounds wherein the response in the presence of the prostaglandin E₂-glyceryl ester antagonist is negligible as compared to the response in the absence of the prostaglandin E₂-glyceryl ester antagonist.

In another aspect of the present invention, the relative activity of a prostaglandin E₂-glyceryl ester agonist may be measured by contacting two or more prostaglandin E₂-glyceryl ester agonists with a tissue or cell that is responsive to a prostaglandin E₂-glyceryl ester agonist in the presence of a specified concentration of a prostaglandin E₂-glyceryl ester antagonist of this invention. The relative activity of each of said prostaglandin E₂-glyceryl ester agonists is determined by comparing the relative response of said tissue or cell.

Those skilled in the art will readily understand that for administration the compounds disclosed herein can be admixed with pharmaceutically acceptable excipients which, per se, are well known in the art. Specifically, a drug to be administered systemically, it may be confected as a powder, pill, tablet or the like, or as a solution, emulsion, suspension, aerosol, syrup or elixir suitable for oral or parenteral administration or inhalation.

For solid dosage forms, non-toxic solid carriers include, but are not limited to, pharmaceutical grades of mannitol, lactose, starch, magnesium stearate, sodium saccharin, the polyalkylene glycols, talcum, cellulose, glucose, sucrose and magnesium carbonate. The solid dosage forms may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the technique described in the U.S. Pat. Nos. 4,256,108; 4,166,452; and 4,265,874 to form osmotic therapeutic tablets for control release. Liquid pharmaceutically administrable dosage forms can, for example, comprise a solution or suspension of one or more of the presently useful compounds and optional pharmaceutical adjutants in a carrier, such as for example, water, saline, aqueous dextrose, glycerol, ethanol and the like, to thereby form a solution or suspension. If desired, the pharmaceutical composition to be administered may also contain minor amounts of nontoxic auxiliary substances such as wetting or emulsifying agents, pH buffering agents and the like. Typical examples of such auxiliary agents are sodium acetate, sorbitan monolaurate, triethanolamine, sodium acetate, triethanolamine oleate, etc. Actual methods of preparing such dosage forms are known, or will be apparent, to those skilled in this art; for example, see Remington's Pharmaceutical Sciences, Mack Publishing Company, Easton, Pa., 16th Edition, 1980. The composition of the formulation to be administered, in any event, contains a quantity of one or more of the presently useful compounds in an amount effective to provide the desired therapeutic effect.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol and the like. In addition, if desired, the injectable pharmaceutical compositions to be administered may also contain minor amounts of non-toxic auxiliary substances such as wetting or emulsifying agents, pH buffering agents and the like. The compounds can also be conjugated to a carrier for topical application to the bone such as a pegylated matrix or a fibrin thrombin matrix.

The amount of the presently useful compound or compounds administered is, of course, dependent on the therapeutic effect or effects desired, on the specific mammal being treated, on the severity and nature of the mammal's condition, on the manner of administration, on the potency and pharmacodynamics of the particular compound or compounds employed, and on the judgement of the prescribing physician. The therapeutically effective dosage of the presently useful compound or compounds is preferably in the range of about 0.5 ng/kg/day or about 1 ng/kg/day to about 100 mg/kg/day.

The compounds of the invention can be administered orally, parenterally, or topically to various mammalian species known to be subject to hyperpigmentary disorders of the skin, hair, internal organs or other pigmented cells or excessive hair growth, e.g., humans, cats, dogs and the like in an effective amount within the dosage range of about 0.1 to about 100 mg/kg, preferably about 0.2 to about 50 mg/kg and more preferably about 0.5 to about 0.5 to about 25 mg/kg (or from about 1 to about 2500 mg, preferably from about 5 to about 2000 mg) on a regimen in single or 2 to 4 divided daily doses. For inflammatory disorders, the compounds of the invention may be given topically, orally, or by local injection, as above.

The active ingredient can be utilized in a composition such as tablet, capsule, solution or suspension containing about 5 to about 500 mg per unit of dosage of a compound or mixture of compounds of formulas I, II or III in topical form for reducing pigmentation or hair growth, etc. (0.01 to 5% by weight compound of formula I, 1 to 5 treatments per day). They may be compounded in conventional matter with a physiologically acceptable vehicle or carrier, excipient, binder, preservative, stabilizer, flavor, etc., or with a topical carrier such as mineral oil as called for by accepted pharmaceutical practice.

The foregoing description details specific methods and compositions that can be employed to practice the present invention, and represents the best mode contemplated. However, it is apparent for one of ordinary skill in the art that further compounds with the desired pharmacological properties can be prepared in an analogous manner, and that the disclosed compounds can also be obtained from different starting compounds. Different pharmaceutical compositions, including the prostaglandin antagonists of this invention, may be prepared and used with substantially the same result.

## Claims

1. A compound having prostaglandin receptor antagonist activity represented by the general formula I. 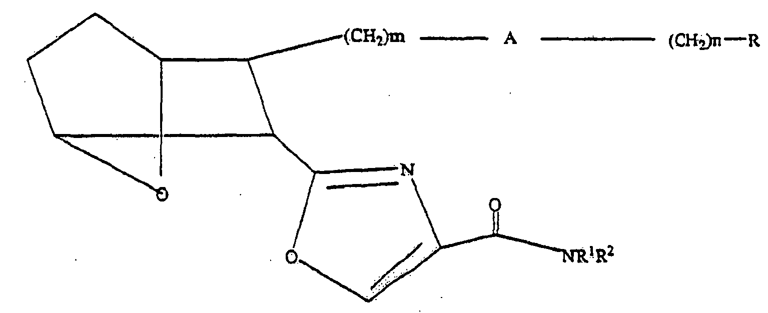
Wherein R is (C(O)-R³- CH₂-CH(OH)(CH₂OH) or -C(O)-R³- CH(CH₂OH)₂ and R³ is selected from the group consisting of O, NR⁴, S and C(R⁵)₂
wherein R⁴ represents a radical selected from the group consisting of H, hydrocarbyl and heteroatom-substituted hydrocarbyl radicals, wherein said hetero atom is selected from the group consisting of halogen, O, S and N, i.e. O, S and/or N may be included as a O, S, or N-containing radical and R⁵ represents a radical selected from the group consisting of H, hydrocarbyl and heteroatom-substituted hydrocarbyl radicals, wherein said hetero atom is selected from the group consisting of halogen, O, S and N, i.e. O, S and/or N may be included as a O, S, or N-containing radical;
m is an integer of from 1 to 3;
n is 0 or an integer of from 1 to 4;
A is an aryl or heteroaryl radical having from 6 to 14 carbon atoms, wherein said heteroaryl may be substituted with one or more oxygen, sulfur or nitrogen in the heteroaryl ring and heteroatom substituted derivatives thereof;
R¹ and R² are independently selected from the group consisting of H, C₁-C₆ alkyl, C₂-C₆ alkenyl, C₂-C₆ alkynyl, C₃-C₇ cycloalkyl, C₄-C₁₂ alkylcycloalkyl, C₆-C₁₀ aryl, C₇-C₁₂ alkylaryl radicals and heteroatom-substituted derivatives thereof, wherein one or more of the hydrogen or carbon atoms in said radicals may be replaced with a halogen, oxygen, nitrogen or sulfur-containing radical; and pharmaceutically acceptable salts thereof.

2. The compounds of claim 1 represented by formula II 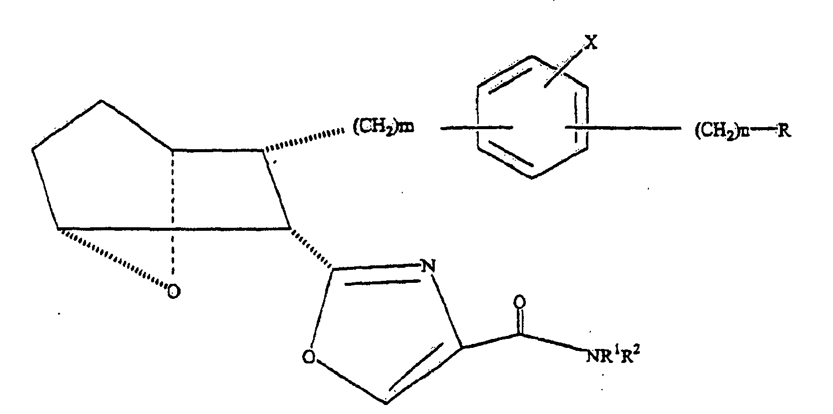 wherein X is selected from the group consisting of H, C₁-C₆ alkyl, hydroxyl, halogen, COOR⁶, NO₂, CF₃, N(R⁶)₂, CON(R⁶)₂, SR⁶, sulfoxy, sulfone, CN and OR⁶, wherein R⁶ is H or C₁-C₆ alkyl.

3. The compounds of claim 2 wherein R¹ and R² are selected from the group consisting of H, C₁-C₆ alkyl, C₃-C₇ cycloalkyl and C₄-C₁₂ alkylcycloalkyl.

4. The compounds of claim 2 wherein X is hydrogen or halogen.

5. The compounds of claim 4 wherein X is fluoro.

6. The compounds of claim 1 represented by formula III 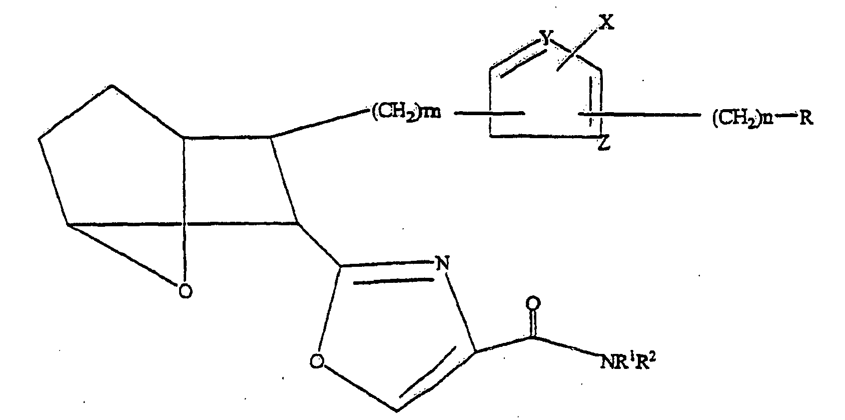 wherein Y is O or S, Z is N or CH and wherein X is selected from the group consisting of H, C₁-C₆ alkyl, hydroxyl, halogen, COOR⁶, NO₂, N(R⁶)₂, CON(R⁶)₂, SR⁶, sulfoxy, sulfone, CN and OR⁶, wherein R⁶ is C₁-C₆ alkyl.

7. The compound of claim 1 or 6 wherein m is 1 or 2.

8. The compound of claim 1 or 6 wherein n is 2 to 4.

9. The compound of claim 6 wherein R¹ .and R² are selected from the group consisting of H, C₁-C₆ alkyl, C₃-C₇ cycloalkyl and C₄-C₁₂ alkylcycloalkyl.

10. The compound of claim 6 wherein X is hydrogen or halogen, sush as fluoro.

11. The compound of claim 1 wherein the compound is 3-(2-{(1R,2R,3S,4R)-3-[4-(4-Cyclohexyl-butylcarbamoyl)-oxazol-2-yl]-7-oxa-bicyclo[22.1]hept-2-ylmethyl}-4-fluoro-phenyl)-propionic acid, 1,3-dihydroxyprop-2-yl ester,
3-(2-{(1R,2R,3S,4R)-3-[4-(4-Cyclohexyl-butylcarbamoyl)-oxazol-2-yl]-7-oxa-bicyclo[2.2.1.]hept-2-ylmethyl}-4-fluoro-phenyl)- 1,3-dihydroxyprop-2-yl-propionamide, or
3-(2-{(1R,2R,3S,4R)-3-[4-(4-Cyclohexyl-butylcarbamoyl)-oxazol-2-yl]-7-oxa-bicyclo[2.2.1]hept-2-ylmethyl}-4-fluoro-phenyl)-propionic acid, 1,2-dihydroxyprop-3-yl ester.

12. The compound according to claim 1 wherein the compound is:

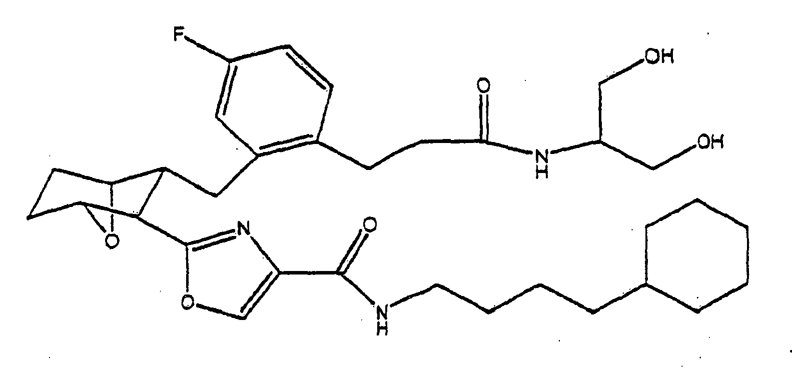

13. Use of a compound according to any one of the preceding claims or a salt thereof for determining compounds having agonist activity at the prostaglandin E receptor.

14. A compound according to any one of claims 1-12 or a salt thereof for use in the treatment or prevention of inflammation and pain in joint and muscle, inflammatory skin condition, inflammatory eye condition, lung disorder in which inflammation is involved, condition of the gastrointestinal tract associated with inflammation, gingivitis, inflammation, pain and tumescence after operation or injury, pyrexia, pain and other conditions associated with inflammation, allergic disease, systemic lupus erythematosus, scleroderma, polymyositis, tendinitis, bursitis, periarteritis nodose, rheumatic fever, Sjogren's syndrome, Behcet disease, thyroiditis, type I diabetes, diabetic complication, nephritic syndrome, aplastic anemia, myasthenia, gravis, uveitis, contact dermatitis, psoriasis, Kawasaki disease, sarcoidosis, Hodgkin's disease, Alzheimer disease, kidney dysfunction, liver dysfunction, gastrointestinal dysfunction shock, bone disease **characterized by** abnormal bone metabolism such as osteoporosis, hyper-calcemia, hyperparathyroidism, Paget's bone diseases, osteolysis, hypercalcemia of malignancy with or without bone metastases, rheumatoid arthritis, periodontitis, osteoarthritis" ostealgia, osteopenia, cancer cachexia, calculosis, lithiasis or solid caricinoma.

## Patentansprüche

1. Verbindung mit Prostaglandinrezeptor-antagonistischer Aktivität, dargestellt durch die allgemeine Formel (1) : 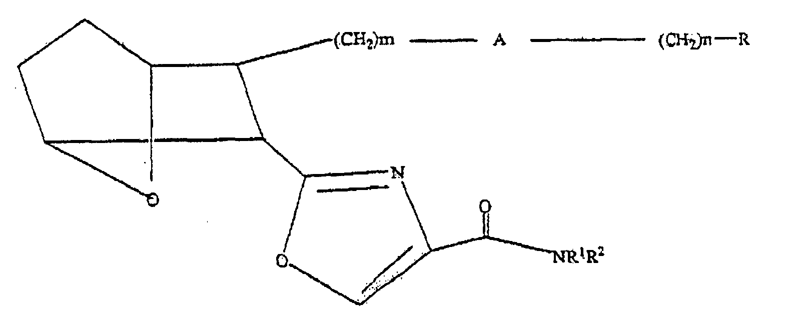 worin:
R -C(O)-R³-CH₂-CH(OH)(CH₂OH) oder -C(O)-R³-CH(CH₂OH)₂ darstellt und R³ aus der Gruppe bestehend aus O, NR⁴, S und C(R⁵)₂ ausgewählt ist, worin R⁴ einen Rest, ausgewählt aus der Gruppe bestehend aus H, Hydrocarbyl und Heteroatom-substituierten Hydrocarbylresten, darstellt, wobei das Heteroatom aus der Gruppe bestehend aus O, S und N ausgewählt ist, d.h. O, S und/oder N können als O, S oder N enthaltender Rest eingeschlossen sein, und R⁵ einen Rest, ausgewählt aus der Gruppe bestehend aus H, Hydrocarbyl und Heteroatom-substituierten Hydrocarbylresten, darstellt, wobei das Heteroatom aus der Gruppe bestehend aus O, S und N ausgewählt ist, d.h. O, S und/oder N können als O, S oder N enthaltender Rest eingeschlossen sein;
m eine ganze Zahl von 1 bis 3 darstellt;
n 0 oder eine ganze Zahl von 1 bis 4 darstellt;
A ein Aryl- oder Heteroarylrest mit 6 bis 14 Kohlenstoffatomen ist, wobei das Heteroaryl mit einem oder mehreren Sauerstoff-, Schwefel- oder Stickstoffatom(en) im Heteroarylring und Heteroatom-substituierten Derivaten davon substituiert sein kann;
R¹ und R² unabhängig aus der Gruppe bestehend aus H, C₁₋₆-Alkyl-, C₂₋₆-Alkenyl-, C₂₋₆-Alkinyl-, C₃₋₇-Cycloalkyl-, C₄₋₁₂-Alkylcycloalkyl-, C₆₋₁₀-Aryl-, C₇₋₁₂-Alkylarylresten und Heteroatom-substituierten Derivaten davon ausgewählt ist, wobei ein oder mehrere Wasserstoff- oder Kohlenstoffatom(e) in dem Rest durch einen Halogen, Sauerstoff, Stickstoff oder Schwefel enthaltenden Rest ersetzt werden kann/können;
und pharmazeutisch annehmbare Salze davon.

2. Verbindung gemäss Anspruch 1, dargestellt durch die Formel (II): 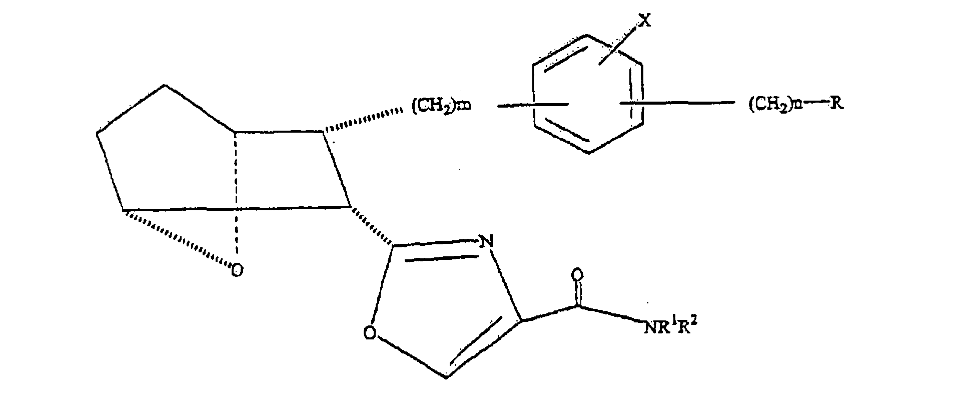 worin X aus der Gruppe bestehend aus H, C₁₋₆-Alkyl, Hydroxyl, Halogen, COOR⁶, NO₂, CF₃, N(R⁶)₂, CON(R⁶)₂, SR⁶, Sulfoxy, Sulfon, CN und OR⁶ ausgewählt ist, worin R⁶ H oder C₁₋₆-Alkyl ist.

3. Verbindung gemäss Anspruch 2, worin R¹ und R² aus der Gruppe bestehend aus H, C₁₋₆-Alkyl, C₃₋₇-Cycloalkyl und C₄₋₁₂-Alkylcycloalkyl ausgewählt sind.

4. Verbindung gemäss Anspruch 2, worin X Wasserstoff oder Halogen ist.

5. Verbindung gemäss Anspruch 4, worin X Fluor ist.

6. Verbindung gemäss Anspruch 1, dargestellt durch die Formel (III): 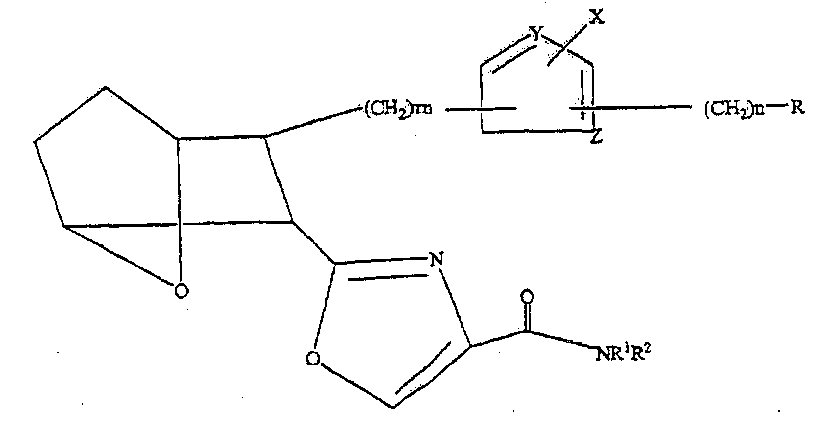 worin Y O oder S ist, Z N oder CH ist und worin X aus der Gruppe bestehend aus H, C₁₋₆-Alkyl, Hydroxyl, Halogen, COOR⁶, NO₂, N(R⁶)₂, CON(R⁶)₂, SR⁶, Sulfoxy, Sulfon, CN und OR⁶ ausgewählt ist, worin R⁶ C₁₋₆-Alkyl ist.

7. Verbindung gemäss Anspruch 1 oder 6, worin m 1 oder 2 ist.

8. Verbindung gemäss Anspruch 1 oder 6, worin n 2 bis 4 ist.

9. Verbindung gemäss Anspruch 6, worin R¹ und R² aus der Gruppe bestehend aus H, C₁₋₆-Alkyl, C₃₋₇-Cycloalkyl und C₄₋₁₂-Alkylcycloalkyl ausgewählt sind.

10. Verbindung gemäss Anspruch 6, worin X Wasserstoff oder Halogen, wie Fluor, ist.

11. Verbindung gemäss Anspruch 1, wobei die Verbindung
3-((2-{(1R,2R,3S,4R)-3-[4-(4-Cyclohexyl-butylcarbamoyl)-oxazol-2-yl]-7-oxabicyclo[2.2.1]hept-2-ylmethyl}-4-fluor-phenyl)-propionsäure-1,3-dihydroxyprop-2-ylester,
3-((2-{(1R,2R,3S,4R)-3-[4-(4-Cyclohexyl-butylcarbamoyl)-oxazol-2-yl]-7-oxa-bicyclo[2.2.1]hept-2-ylmethyl}-4-fluor-phenyl)-1,3-dihydroxyprop-2-yl-propionamid oder 3-(2-{(1R,2R,3S,4R)-3-[4-(4-Cyclohexyl-butylcarbamoyl)-oxazol-2-yl]-7-oxa-bicyclo[2.2.1]hept-2-ylmethyl}-4-fluor-phenyl)-propionsäure-1,2-dihydroxyprop-3-ylester
ist.

12. Verbindung gemäss Anspruch 1, wobei die Verbindung 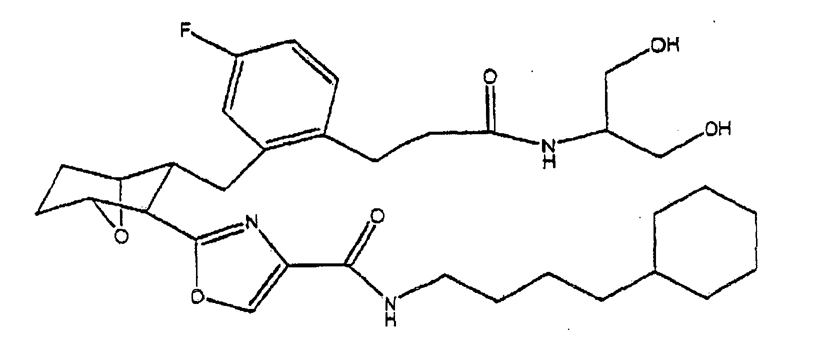 ist.

13. Verwendung einer Verbindung gemäss irgendeinem der vorhergehenden Ansprüche oder eines Salzes davon zur Bestimmung von Verbindungen, die agonistische Aktivität am Prostaglandin E-Rezeptor besitzen.

14. Verbindung gemäss irgendeinem der Ansprüche 1 bis 12 oder ein Salz davon zur Verwendung bei der Behandlung oder Prävention von Entzündungen und Schmerzen in Gelenken und Muskeln, entzündlichen Hauterkrankungen, entzündlichen Augenerkrankungen, Lungenerkrankung, an der eine Entzündung beteiligt ist, Beschwerden des Magen-Darm-Trakts, die mit Entzündungen verbunden sind, Zahnfleischentzündung, Entzündung, Schmerz und Schwellung nach einer Operation oder Verletzung, Fieber, Schmerzen und anderen Beschwerden, die mit Entzündungen verbunden sind, allergischen Erkrankungen, systemischem Lupus erythematodes, Sklerodermie, Polymyositis, Tendinitis, Bursitis, Periarteriitis nodosa, rheumatischem Fieber, Sjögren-Syndrom, Behcet-Krankheit, Thyreoiditis, Typ I-Diabetes, diabetischen Komplikationen, nephrotischem Syndrom, aplastischer Anämie, Muskelschwäche, Gravis, Uveitis, Kontaktdermatitis, Psoriasis, Kawasaki-Krankheit, Sarkoidose, Morbus Hodgkin, Alzheimer-Krankheit, Nierenfunktionsstörungen, Leberfunktionsstörungen, Magen-Darm-Funktionsstörungen, Schock, Knochenerkrankungen, die durch einen abnormen Knochenstöffwechsel gekennzeichnet sind, wie Osteoporose, Hyperkalzämie, Hyperparathyreoidismus, Paget-Knochenerkrankungen, Osteolyse, durch Malignome bedingte Hyperkalzämie mit oder ohne Knochenmetastasen, rheumatoider Arthritis, Paradontitis, Osteoarthritis, Ostealgie, Osteopenie, Tumorkachexie, Steinleiden, Lithiase oder soliden Karzinomen.

## Revendications

1. Composé ayant une activité d'antagoniste de récepteur de prostaglandine, représenté par la formule générale I : 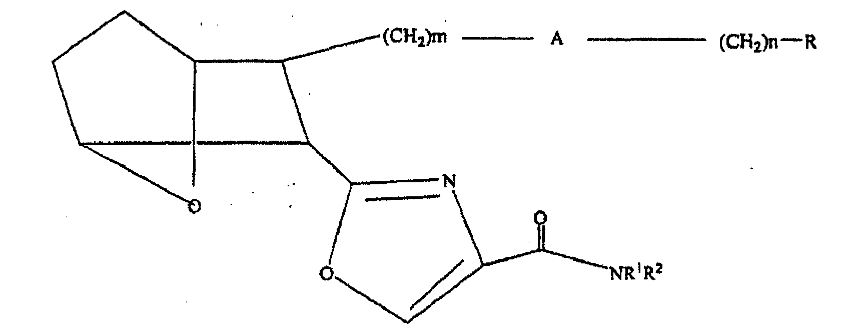
dans laquelle R est -C(O)-R³-CH₂-CH(OH)(CH₂OH) ou -C(O)-R³-CH(CH₂OH)₂ et R³ est choisi dans le groupe constitué par O, NR⁴, S et C(R⁵)₂ ;
où R⁴ représente un radical choisi dans le groupe constitué par H et les radicaux hydrocarbyle et hydrocarbyle substitués par un hétéroatome, ledit hétéroatome étant choisi dans le groupe constitué par les halogènes, O, S et N, c'est-à-dire que O, S et/ou N peuvent être inclus sous la forme d'un radical contenant O, S ou N, et R⁵ représente un radical choisi dans le groupe constitué par H et les radicaux hydrocarbyle et hydrocarbyle substitués par un hétéroatome, ledit hétéroatome étant choisi dans le groupe constitué par les halogènes, O, S et N, c'est-à-dire que O, S et/ou N peuvent être inclus sous la forme d'un radical contenant O, S ou N ;
m est un entier de 1 à 3 ;
n vaut 0 ou est un entier de 1 à 4 ;
A est un radical aryle ou hétéroaryle ayant de 6 à 14 atomes de carbone, ledit hétéroaryle pouvant être substitué par un ou plusieurs atomes d'oxygène, de soufre ou d'azote dans le cycle hétéroaryle et ses dérivés substitués par un hétéroatome ;
R¹ et R² sont indépendamment choisis dans le groupe constitué par H et les radicaux alkyle en C₁ à C₆, alcényle en C₂ à C₆, alcynyle en C₂ à C₆, cycloalkyle en C₃ à C₇, alkylcycloalkyle en C₄ à C₁₂, aryle en C₆ à C₁₀, arylalkyle en C₇ à C₁₂ et leurs dérivés substitués par un hétéroatome, un ou plusieurs des atomes d'hydrogène ou de carbone dans lesdits radicaux pouvant être remplacés par un radical halogéné, oxygéné, azoté ou soufré ;
et ses sels pharmaceutiquement acceptables.

2. Composés selon la revendication 1, représentés par la formule II : 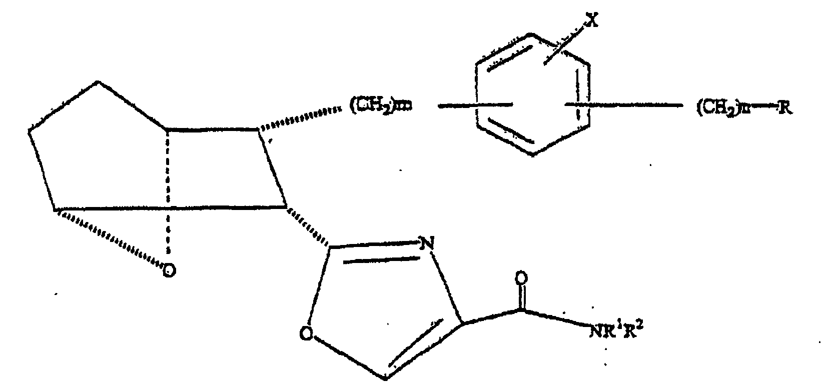 dans laquelle X est choisi dans le groupe constitué par H, les halogènes, alkyle en C₁ à C₆, hydroxyle, COOR⁶, NO₂, CF₃, N(R⁶)₂, CON(R⁶)₂, SR⁶, sulfoxy, sulfone, CN et OR⁶, où R⁶ est H ou un alkyle en C₁ à C₆.

3. Composés selon la revendication 2, dans lesquels R¹ et R² sont choisis dans le groupe constitué par H, alkyle en C₁ à C₆, cycloalkyle en C₃ à C₇ et alkylcycloalkyle en C₄ à C₁₂.

4. Composés selon la revendication 2, dans lesquels X est l'hydrogène ou un halogène.

5. Composés selon la revendication 4, dans lesquels X est fluoro.

6. Composés selon la revendication 1, représentés par la formule III : 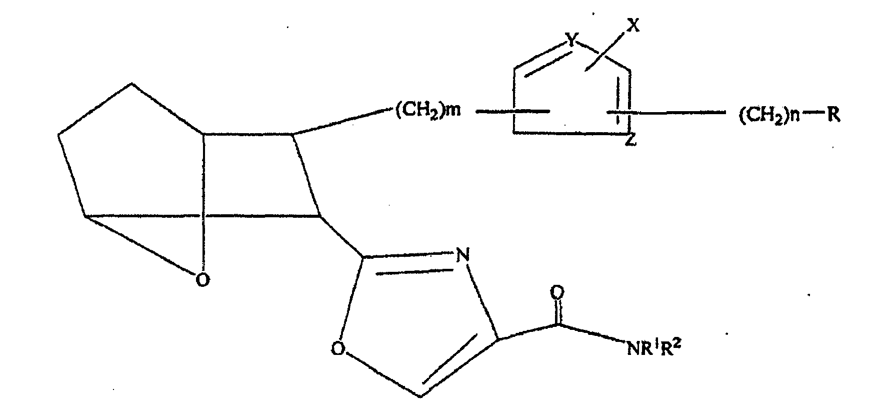 dans laquelle Y est O ou S, Z est N ou CH, et X est choisi dans le groupe constitué par H, les halogènes, alkyle en C₁ à C₆, hydroxyle, COOR⁶, NO₂, N(R⁶)₂, CON(R⁶)₂, SR⁶, sulfoxy, sulfone, CN et OR⁶, où R⁶ est un alkyle en C₁ à C₆.

7. Composé selon la revendication 1 ou 6, dans lequel m vaut 1 ou 2.

8. Composé selon la revendication 1 ou 6, dans lequel n vaut de 2 à 4.

9. Composé selon la revendication 6, dans lequel R¹ et R² sont choisis dans le groupe constitué par H, alkyle en C₁ à C₆, cycloalkyle en C₃ à C₇ et alkylcycloalkyle en C₄ à C₁₂.

10. Composé selon la revendication 6, dans lequel X est l'hydrogène ou un halogène tel que fluoro.

11. Composé selon la revendication 1, lequel composé est l'ester 1,3-dihydroxyprop-2-ylique d'acide 3-(2-{(1R,2R,3S,4R)-3-[4-(4-cyclohexylbutylcarbamoyl)oxazol-2-yl]-7-oxabicyclo[2.2.1]hept-2-ylméthyl}-4-fluorophényl)-propionique, le 3-(2-{(1R,2R,3S,4R)-3-[4-(4-cyclohexyl-butylcarbamoyl)oxazol-2-yl]-7-oxabicyclo[2.2.1]hept-2-ylméthyl}-4-fluorophényl)-1,3-dihydroxyprop-2-yl-propionamide ou l'ester 1,2-dihydroxyprop-3-ylique d'acide 3-(2-{(1R,2R,3S,4R)-3-[4-(4-cyclohexylbutylcarbamoyl)oxazol-2-yl]-7-oxabicyclo-[2.2.1]hept-2-ylméthyl}-4-fluorophényl)-propionique.

12. Composé selon la revendication 1, lequel composé est :

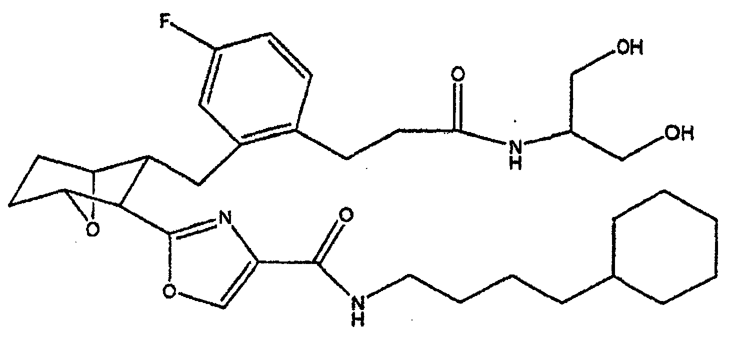

13. Utilisation d'un composé selon l'une quelconque des revendications précédentes ou d'un sel de celui-ci pour déterminer des composés ayant une activité d'agoniste au niveau du récepteur de prostaglandine E.

14. Composé selon l'une quelconque des revendications 1 à 12 ou sel de celui-ci pour utilisation dans le traitement ou la prévention d'une inflammation et d'une douleur dans une articulation et un muscle, d'un état cutané inflammatoire, d'un état oculaire inflammatoire, d'un trouble pulmonaire dans lequel une inflammation est impliquée, d'un état des voies gastro-intestinales associé à une inflammation, d'une gingivite, d'une inflammation, d'une douleur et d'une tumescence après opération ou blessure, d'une pyrexie, d'une douleur et d'autres états associés à une inflammation, d'une maladie allergique, d'un lupus érythémateux disséminé, d'une sclérodermie, d'une polymyosite, d'une tendinite, d'une bursite, d'une périartérite noueuse, d'un rhumatisme articulaire aigu, d'un syndrome de Sjôgren, d'une maladie de Behcet, d'une thyroïdite, d'un diabète de type I, d'une complication diabétique, d'un syndrome néphritique, d'une anémie aplasique, d'une myasthénie grave, d'une uvéite, d'une dermatite de contact, d'un psoriasis, d'une maladie de Kawasaki, d'une sarcoïdose, d'une maladie de Hodgkin, d'une maladie d'Alzheimer, d'un dysfonctionnement rénal, d'un dysfonctionnement hépatique, d'un choc de dysfonctionnement gastro-intestinal, d'une maladie osseuse **caractérisée par** un métabolisme osseux anormal telle qu'une ostéoporose, une hypercalcémie, une hyperparathyroïdie, des maladies osseuses de Paget, une ostéolyse, une hypercalcémie de malignité avec ou sans métastases osseuses, une polyarthrite rhumatoïde, une parodontie, une arthrose, une ostéalgie, une ostéopénie, d'une cachexie liée à un cancer, d'une calculose, d'une lithiase ou d'un carcinome solide.
